(19) Europäisches Patentamt / European Patent Office / Office européen des brevets

(11) **EP 1 108 438 B1**

(12) **FASCICULE DE BREVET EUROPEEN**

(45) Date de publication et mention
de la délivrance du brevet:
**18.12.2013   Bulletin 2013/51**

(51) Int Cl.:
***A61M 1/16*** *(2006.01)*

(21) Numéro de dépôt: **00204121.8**

(22) Date de dépôt: **21.11.2000**

(54) **Procédé de détermination d'un paramètre significatif du progrès d'un traitement extracorporel de sang**

Verfahren zur Bestimmung eines Parameters, der für den Verlauf einer extrakorporalen Blutbehandlung von Bedeutung ist

Process for determining a parameter significative of the progress of an extracorporeal blood treatment

(84) Etats contractants désignés:
**AT BE CH CY DE DK ES FI FR GB GR IE IT LI LU MC NL PT SE TR**

(30) Priorité:  **02.12.1999   FR 9915196**

(43) Date de publication de la demande:
**20.06.2001   Bulletin 2001/25**

(73) Titulaire: **GAMBRO HOSPAL (Schweiz) AG
4051 Basel (CH)**

(72) Inventeurs:
• **Bosetto, Antonio
41037 Mirandola (IT)**
• **Paolini, Francesco
41010 Modena (IT)**

(74) Mandataire: **Ponzellini, Gianmarco et al
Gambro Intellectual Property Dept.
P.O. Box 98
41037 Mirandola (IT)**

(56) Documents cités:
EP-A- 0 504 725      EP-A- 0 658 352
WO-A-93/00938      WO-A-98/32476

**Description**

L'invention concerne un dispositif de détermination d'un paramètre significatif du progrès d'un traitement extracorporel de sang, en particulier d'un traitement d'épuration ayant pour but de pallier l'insuffisance rénale, comme l'hémodialyse ou l'hémodiafiltration.

Pour mémoire, l'hémodialyse consiste à faire circuler, de part et d'autre de la membrane semi-perméable d'un hémodialyseur, le sang d'un patient et un liquide de traitement sensiblement isotonique au sang, de façon que, lors du transfert diffusif qui s'établit au travers de la membrane pour les substances ayant des concentrations différentes de part et d'autre de la membrane, les impuretés du sang (urée, créatinine, etc.) migrent du sang vers le liquide de traitement. La concentration ionique du liquide de traitement est aussi généralement choisie pour corriger la concentration ionique du sang du patient.

Dans le traitement par hémodiafiltration, au transfert diffusif obtenu par dialyse s'ajoute un transfert convectif par ultrafiltration résultant d'une différence de pression positive créée entre le coté sang et le coté liquide de traitement de la membrane.

Il est du plus grand intérêt de pouvoir déterminer, tout au long d'une séance de traitement, un ou plusieurs paramètres significatifs du progrès du traitement afin de pouvoir, le cas échéant, modifier les conditions du traitement telles qu'elles ont été fixées initialement en vue d'un objectif thérapeutique déterminé.

Les paramètres dont la connaissance permet de suivre le progrès du traitement, c'est-à-dire aussi d'apprécier l'adéquation des conditions de traitement fixées initialement à l'objectif thérapeutique, sont, en particulier, la concentration du sang en un soluté donné (sodium par exemple), ou la dialysance D réelle ou la clairance K réelle de l'échangeur pour tel ou tel soluté (la dialysance D et la clairance K représentant le rendement épuratif de l'échangeur) ou la dose de dialyse administrée après un temps t de traitement, laquelle, d'après les travaux de Sargent et Gotch, peut être assimilée au rapport sans dimension Kt/V, où K est la clairance K réelle pour l'urée, t le temps de traitement écoulé, et V le volume de distribution de l'urée, c'est-à-dire le volume d'eau total du patient (Gotch FA, Sargent SA. A mechanistic analysis of the National Cooperative Dialysis Study (NCDS). Kidney int 1985 ; 28 : 526-34).

Ces paramètres posent tous le même problème pour leur détermination, qui est de nécessiter la connaissance précise d'une caractéristique physique ou chimique du sang, alors que cette caractéristique ne peut pas, dans la pratique, être obtenue par mesure directe sur un échantillon pour des raisons thérapeutiques, prophylactiques et pécuniaires: en premier lieu, il est exclu de prélever sur un patient, souvent anémié, les échantillons multiples qui seraient nécessaires pour suivre l'efficacité du traitement au cours de son déroulement; en outre, compte tenu des risques liés à la manipulation d'échantillons de sang éventuellement contaminé, la tendance générale est à éviter de telles manipulations ; enfin, l'analyse d'un échantillon de sang en laboratoire est à la fois coûteuse et relativement longue, ce qui est incompatible avec l'objectif recherché.

Plusieurs procédés ont été proposés à ce jour pour déterminer *in vivo* des paramètres de l'hémodialyse sans avoir à effectuer de mesures sur le sang.

Le document EP 0 547 025 décrit un procédé pour déterminer la concentration d'une substance, telle que le sodium, dans le sang d'un patient soumis à un traitement d'hémodialyse. Ce procédé, qui permet aussi de déterminer la dialysance D - par exemple pour le sodium - de l'hémodialyseur utilisé pour administrer le traitement, comprend les étapes de:

- faire circuler successivement dans l'hémodialyseur un premier et un second liquides d'hémodialyse ayant des concentrations différentes en sodium;
- mesurer la conductivité du premier et du second liquides de dialyse, en amont et en aval de l'hémodialyseur; et
- calculer la concentration du sodium dans le sang du patient (ou la dialysance D de l'hémodialyseur pour le sodium) à partir des valeurs de la conductivité du liquide mesurées dans le premier et le second liquides de dialyse en amont et en aval de l'hémodialyseur.

Le document EP 0 658 352 décrit un autre procédé pour la détermination *in vivo* des paramètres de l'hémodialyse, comprenant les étapes de:

- faire circuler successivement dans l'hémodialyseur au moins un premier et un second liquides de traitement ayant une caractéristique (conductivité, par exemple) liée à au moins un des paramètres significatifs du traitement (concentration ionique du sang, dialysance D, clairance K, Kt/V, par exemple), la valeur de la caractéristique dans le premier liquide en amont de l'échangeur étant différente de la valeur de la caractéristique dans le second liquide en amont de l'échangeur,
- mesurer dans chacun des premier et second liquides de traitement deux valeurs de la caractéristique, respectivement en amont et en aval de l'échangeur ;
- mettre en circulation un troisième liquide de traitement dans l'échangeur alors que la caractéristique du second liquide n'a pas atteint une valeur stable en aval de l'échangeur, la valeur de la caractéristique dans le troisième liquide en amont de l'échangeur étant différente de la valeur de la caractéristique dans le second liquide en amont de l'échangeur,

- mesurer deux valeurs de la caractéristique dans le troisième liquide respectivement en amont et en aval de l'échangeur, et
- calculer au moins une valeur d'au moins un paramètre significatif du progrès du traitement à partir des valeurs mesurées de la caractéristique dans le premier, le second, et le troisième liquides de traitement.

Un autre procédé pour la détermination *in vivo* des paramètres de l'hémodialyse ne nécessitant pas d'effectuer des mesures sur le sang est décrit dans le document EP 0 920 877. Ce procédé comporte les étapes de :

- faire circuler dans l'échangeur un liquide de traitement ayant une caractéristique ayant une valeur nominale sensiblement constante en amont de l'échangeur;
- faire varier la valeur de la caractéristique en amont de l'échangeur puis rétablir la caractéristique à sa valeur nominale en amont de l'échangeur;
- mesurer et mettre en mémoire une pluralité de valeurs prises par la caractéristique du liquide de traitement en aval de l'échangeur en réponse à la variation de la valeur de cette caractéristique provoquée en amont de l'échangeur;
- déterminer la superficie d'une zone de perturbation aval délimitée par une ligne de base et une courbe représentative de l'évolution par rapport au temps de la caractéristique; et
- calculer le paramètre significatif de l'efficacité d'un traitement à partir de la superficie de la zone de perturbation aval et de la superficie d'une zone de perturbation amont délimitée par une ligne de base et une courbe représentative de l'évolution par rapport au temps de la caractéristique en amont de l'échangeur.

Tous ces procédés ont pour point commun de comprendre une modification momentanée de la valeur d'une caractéristique du liquide de dialyse (la conductivité, par exemple) puis le rétablissement de cette caractéristique à sa valeur initiale, qui est généralement la valeur de prescription. Même si le séquencement des mesures est tel que la détermination du paramètre recherché peut prendre moins de deux minutes (cas du deuxième procédé mentionné), il reste que tous ces procédés ne peuvent être mis en oeuvre en pratique que six fois par heure.

Un but de l'invention est de proposer un dispositif de détermination d'un paramètre significatif du progrès d'un traitement extracorporel de sang qui soit quasi continu, fiable et sans influence sur le traitement mis en oeuvre.

Pour atteindre ce but, on prévoit un dispositif selon la revendication 1.

Ce dispositif présente l'intérêt de permettre une détermination précise et continue de paramètres significatifs du progrès du traitement à partir de mesures effectuées en continu. Le patient n'est jamais exposé à un liquide de traitement très différent du liquide de traitement prescrit (par exemple trop riche ou trop pauvre en sodium). Par ailleurs, ce dispositif est peu sensible aux incidents de tous ordres qui peuvent se produire au moment de la mesure d'une valeur isolée et qui peuvent fausser les calculs ultérieurs faisant intervenir une valeur erratique.

La mise en oeuvre de ce dispositif de détermination continue peut être réalisée selon une ou plusieurs des modalités spécifiques suivantes:

- le paramètre (D, Cbin, K, Kt/v) significatif de l'efficacité du traitement extracorporel du sang est calculé chaque fois qu'une nouvelle valeur (Cdoutj) de la caractéristique (Cd) en aval de l'échangeur (1) à été mémorisée;
- la deuxième série de valeurs (Cdoutj) de la caractéristique (Cd) en aval de l'échangeur (1) comprend la dernière valeur mise en mémoire;
- la deuxième série de valeurs (Cdoutj) de la caractéristique (Cd) en aval de l'échangeur (1) comprend un nombre déterminé de valeurs successives.

Selon une caractéristique de l'invention, une correspondance est établie entre chaque valeur (Cdoutj+z) de la deuxième série de valeurs et une valeur (Cdinj) de la première série de valeur, la valeur (Cdoutj+z) de la deuxième série de valeurs étant décalée dans le temps par rapport à la valeur (Cdinj) correspondante de la première série de valeurs d'un délai hydraulique (T) égal au temps mis par un échantillon de liquide pour s'écouler dans un circuit de liquide de traitement connecté à l'échangeur, entre un point situé en amont de l'échangeur et un point situé en aval de l'échangeur.

Lorsque le délai hydraulique (T) est l'un des coefficients du modèle mathématique, il peut être déterminé par:

- calcul au moyen du modèle mathématique, pour chaque valeur (Cdinj) de la première série de valeurs, une valeur (Cd*outj+z) correspondante de la caractéristique (Cd) en aval de l'échangeur; et
- détermination de la valeur optimale du délai hydraulique (T) pour laquelle la correspondance entre les valeurs calculées (Cd*outj+z) de la caractéristique (Cd) en aval de l'échangeur et les valeurs mesurées (Cdoutj+z) correspondantes de la caractéristique (Cd) en aval de l'échangeur est la plus exacte.

Selon uneautre caractéristique de l'invention, le calcul d'un paramètre (D, Cbin) significatif de l'efficacité du traitement extracorporel du sang comprend les étapes de:

- calcul au moyen du modèle mathématique, pour chaque valeur (Cdinj) de la première série de valeurs, une valeur (Cd*outj+z) correspondante de la caractéristique (Cd) en aval de l'échangeur; et
- détermination de la valeur optimale du paramètre (D, Cbin) pour laquelle la correspondance entre les valeurs calculées (Cd*outj+z) de la caractéristique (Cd) en aval de l'échangeur et les valeurs mesurées (Cdoutj+z) correspondantes de la caractéristique (Cd) en aval de l'échangeur est la plus exacte.

Selon encore une autre caractéristique de l'invention, le modèle mathématique est linéaire et la détermination de la valeur optimale du paramètre (D, Cbin) consiste à déterminer la valeur du paramètre (D, Cbin) pour laquelle la somme des carrés des différences entre valeurs mesurées (Cdoutj+z) et valeurs calculées (Cd*outj+z) correspondantes de la caractéristique (Cd) en aval de l'échangeur est la moindre.

Conformément à l'invention, la variation de la caractéristique (Cd) en amont de l'échangeur peut être réalisée selon un des modes de réalisation suivantes:

- ou bien la caractéristique est réglée en continu en fonction de l'évolution d'un paramètre d'un dispositif destiné à la mise en oeuvre du traitement et/ou d'un paramètre du patient (par exemple, la variation relative du volume du sang du patient), de façon que ce paramètre reste contenu à l'intérieur d'une fourchette de valeurs admissibles.
- ou bien la caractéristique est réglée conformément à une règle de variation préalablement mise en mémoire, prévoyant, par exemple, l'alternance régulière d'une augmentation et d'une diminution de la caractéristique, d'une quantité déterminée.
- ou bien la caractéristique fluctue au gré de perturbations inhérentes à la préparation du liquide de traitement.

D'autres caractéristiques et avantages de l'invention apparaîtront à la lecture de la description qui suit. On se reportera à la figure unique qui représente de façon schématique et partielle un système d'hémodialyse et d'hémodiafiltration adapté à la mise en oeuvre du dispositif selon l'invention.

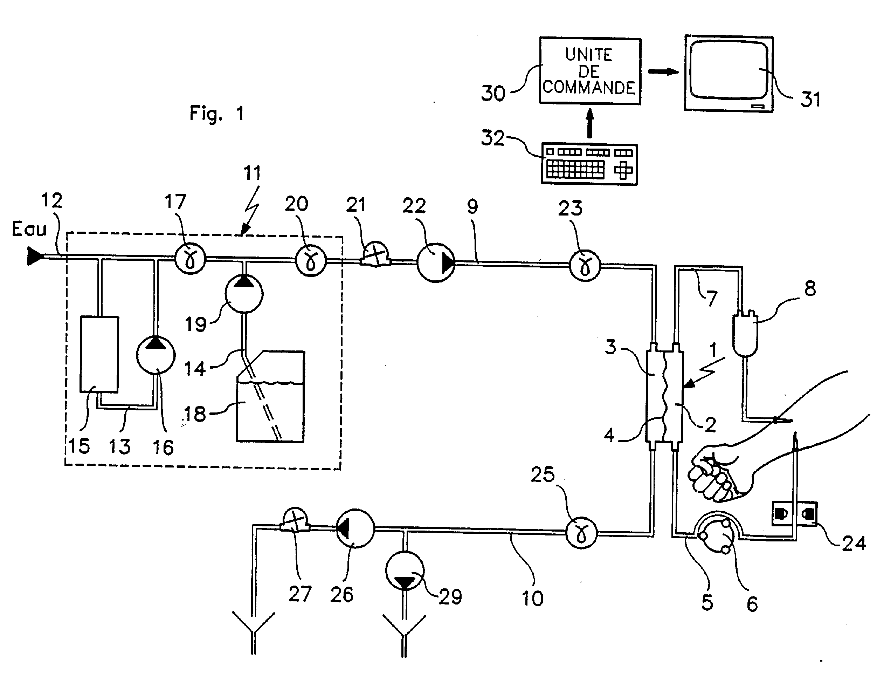

Le système d'hémodialyse représenté sur la figure 1 comprend un hémodialyseur 1 ayant deux compartiments 2, 3 séparés par une membrane semi-perméable 4. Un premier compartiment 2 a une entrée connectée à une canalisation 5 de prélèvement de sang sur laquelle sont disposées une sonde 24 de mesure d'hémoglobine et une pompe de circulation 6, et une sortie connectée à une canalisation 7 de restitution de sang sur laquelle est interposé un piège à bulles 8.

Le second compartiment 3 de l'hémodialyseur 1 a une entrée connectée à une canalisation 9 d'alimentation en liquide de dialyse frais et une sortie connectée à une canalisation 10 d'évacuation de liquide usé (liquide de dialyse et ultrafiltrat).

La canalisation d'alimentation 9 relie l'hémodialyseur 1 à un dispositif de préparation de liquide de dialyse 11 comprenant une canalisation principale 12 dont l'extrémité amont est prévue pour être raccordée à une source d'eau courante. A cette canalisation principale 12 sont connectées une première et une deuxième canalisation secondaires 13, 14. La première canalisation secondaire 13, qui est rebouclée sur la canalisation principale 12, est munie de moyens de raccord pour le montage d'une cartouche 15 contenant du bicarbonate de sodium sous forme de granulés. Elle est en outre équipée d'une pompe 16 de dosage du bicarbonate dans le liquide de dialyse, disposée en aval de la cartouche 15. La pompe 16 est pilotée en fonction de la comparaison entre 1) une première valeur de consigne de conductivité pour la solution se formant à la jonction de la canalisation principale 12 et de la canalisation secondaire 13 et 2) la valeur de la conductivité de ce mélange mesurée au moyen d'une première sonde de conductivité 17 disposée sur la canalisation principale 12 immédiatement en aval de la jonction entre la canalisation principale 12 et la première canalisation secondaire 13.

L'extrémité libre de la deuxième canalisation secondaire 14 est destinée à être immergée dans un conteneur 18 pour une solution saline concentrée contenant du chlorure de sodium, de calcium, de magnésium et de potassium, ainsi que de l'acide acétique. La seconde canalisation 14 est équipée d'une pompe 19 de dosage du sodium dans le liquide de dialyse qui est pilotée en fonction de la comparaison entre 1) une seconde valeur de consigne de conductivité pour la solution se formant à la jonction de la canalisation principale 12 et de la seconde canalisation secondaire 14 et 2) la valeur de la conductivité de cette solution mesurée au moyen d'une deuxième sonde de conductivité 20 disposée sur la canalisation principale 12 immédiatement en aval de la jonction entre la canalisation principale 12 et la canalisation secondaire 14.

La canalisation d'alimentation 9 forme le prolongement de la canalisation principale 12 du dispositif 11 de préparation de liquide de dialyse. Sur cette canalisation d'alimentation sont disposés, dans le sens de circulation du liquide, un premier débitmètre 21, une première pompe de circulation 22 et une troisième sonde de conductivité 23.

L'extrémité aval de la canalisation 10 d'évacuation de liquide usé est prévue pour être reliée à l'égout. Sur cette canalisation sont disposés, dans le sens de circulation du liquide, une quatrième sonde de conductivité 25, une deuxième pompe de circulation 26 et un deuxième débitmètre 27. Une pompe d'extraction 29 est reliée à la canalisation d'évacuation 10, en amont de la deuxième pompe de circulation 26.

Le système d'hémodialyse représenté sur la figure 1 comprend également une unité de calcul et de commande 30. Cette unité est reliée à un écran 31 et à un clavier 32 par lequel l'utilisateur lui communique diverses valeurs de consigne : consignes de débit (débit de sang Qb, débit de liquide de dialyse Qd), consignes de conductivité utilisées pour la préparation du liquide de dialyse, consigne de durée de traitement, consigne de perte de poids WL. Par ailleurs, l'unité de calcul et de commande 30 reçoit des information émises par les organes de mesure du système, tels que les débitmètres 21, 27, les sondes de conductivité 17, 20, 23, 25, la sonde 24 de mesure d'hémoglobine. Elle pilote, en fonction des instructions reçues et de modes d'opération et d'algorithmes programmés les organes moteurs du système, tels que les pompes 6, 16, 19, 22, 26, 29.

Le système d'hémodialyse qui vient d'être décrit peut fonctionner selon un premier mode, relativement simple, et un second mode, plus sophistiqué.

Premier mode de fonctionnement:

Après que le circuit extracorporel de sang a été rincé et rempli de solution saline stérile, il est connecté au patient et, la pompe à sang 6 est mise en service à un débit prédéterminé Qb, 200 ml/min. par exemple.

Simultanément, les pompes 16 et 19 du dispositif de préparation de liquide de dialyse 11, les pompes de circulation de liquide de dialyse 22, 26 et la pompe d'extraction 29 sont mises en service. Le débit des pompes de dosage 16, 19 est réglé au moyen des sondes de conductivité 17, 20 pour que le liquide de dialyse ait la concentration en bicarbonate et la concentration en sodium souhaitées. Le débit Qd de la pompe de circulation 22 disposée sur la canalisation d'alimentation 9 est réglé à une valeur fixe (500 ml/min., par exemple), tandis que le débit de la pompe de circulation 26 disposée sur la canalisation d'évacuation 10 est ajusté en permanence de façon que le débit mesuré par le second débitmètre 27 soit égal au débit mesuré par le premier débitmètre 21. Le débit de la pompe d'extraction 29 est réglé pour être égal au débit de perte de poids (calculé à partir du poids WL qu'il est prescrit de faire perdre au patient et de la durée de la séance de traitement) augmenté éventuellement du débit d'un liquide perfusé au patient.

Le signal délivré par la sonde 24 de mesure d'hémoglobine est utilisé par l'unité de commande 30 pour calculer régulièrement, à partir de la valeur initiale de la concentration du sang en hémoglobine, les variations relatives du volume du sang du patient.

Second mode de fonctionnement:

Dans le second mode de fonctionnement, l'unité de commande 30 commande en outre la pompe d'extraction 29 et/ou la pompe la pompe de dosage 19 de façon que les variations relatives du volume du sang du patient restent dans un domaine de valeurs admissibles.

L'efficacité du traitement administré au patient au moyen du système qui vient d'être décrit est déterminé en continu au moyen du dispositif suivant, dont la mise en oeuvre suppose la définition préalable d'un modèle mathématique traduisant, sous la forme d'une équation ou d'un système d'équations, l'influence d'une caractéristique (Cd) du liquide de dialyse sur l'efficacité du traitement, ce modèle mathématique ayant au moins un coefficient constitué par un paramètre (D, Cbin) significatif de l'efficacité du traitement extracorporel du sang.

Dans ce qui suit on prendra l'exemple d'un modèle mathématique des échanges se produisant au travers de la membrane 4 de l'hémodialyseur 1. Ce modèle mathématique, dont au moins un des coefficients est l'un des paramètres significatif de l'efficacité du traitement que l'on cherche à déterminer, établit une relation entre une valeur d'une caractéristique d'un volume élémentaire du liquide de dialyse en amont de l'hémodialyseur 1 et une valeur de la caractéristique d'un volume élémentaire en aval de l'hémodialyseur 1. Ainsi, un modèle mathématique mettant en relation la valeur Cdin de la concentration ionique (ou de la conductivité) d'un échantillon de liquide de dialyse en amont de l'hémodialyseur avec la valeur Cdout de la concentration ionique (ou de la conductivité) d'un échantillon de liquide de dialyse en aval de l'hémodialyseur peut comprendre, par exemple, un ou plusieurs des coefficients suivants:

- la dialysance D,
- la concentration ionique du sang Cbin,
- le délai hydraulique T, qui est égal au temps mis par un échantillon de liquide pour s'écouler entre le point de mesure amont de la conductivité (la deuxième sonde 20 de conductivité, si les valeurs de conductivité utilisées par l'unité de calcul 30 sont les valeurs de consigne, ou la troisième sonde 23 de conductivité, si les valeurs de conductivité utilisées par l'unité de calcul 30 sont des valeurs mesurées) et le point de mesure aval de la conductivité (la quatrième sonde de conductivité 25); le délai hydraulique T dépend essentiellement du débit de liquide de dialyse, du volume des canalisations 9 et 10 entre les sondes de conductivité 20 ou 23 et 25 et de la contenance du compartiment 3 de liquide de dialyse de l'hémodialyseur 1, et

- la constante de temps ø du système; la constante de temps ne dépend que du débit du liquide de dialyse, du débit du sang, de la surface de la membrane 4 et du coefficient de diffusion de la membrane pour le soluté considéré, c'est-à-dire ici le sodium.

Une fois défini, le modèle mathématique est mis en mémoire dans une mémoire de l'unité de commande et de calcul 30.

Le procédé mise en oeuvre par le dispositif selon l'invention comprend une première étape par laquelle la conductivité du liquide de dialyse en amont de l'hémodialyseur 1 est soumise, de préférence pendant toute la séance de traitement, à une succession de variations de faible amplitude (c'est-à-dire ne s'écartant pas, ou rarement, de plus de environ 5% de la conductivité moyenne du liquide de dialyse). Cette succession de variations peut être commandée ou incontrôlée.

Elle est incontrôlée quand, par exemple, la commande des pompes 16 et 19 n'est pas parfaitement asservie aux mesures effectuées par les sondes de conductivité 17 et 20 et, que le liquide de dialyse produit le générateur 11 n'est pas tout à fait homogène.

La succession des variations est commandée quand, par exemple, elle suit une règle de variation prédéterminée, mise en mémoire dans une mémoire de l'unité de contrôle et de calcul 30: la vitesse de la pompe 19 peut, par exemple, soit être modifiée de façon aléatoire, soit être modifiée régulièrement pour que la conductivité du liquide de dialyse augmente puis diminue en continu pendant le même temps d'une même quantité au delà et en deçà de la valeur de prescription.

La succession des variations est commandée aussi quand, conformément au deuxième mode de fonctionnement du système d'hémodialyse mentionné plus haut, la concentration en sodium du liquide de dialyse est asservie à une comparaison entre la variation relative mesurée du volume du sang et un domaine de valeurs admissibles.

Dans une deuxième étape du procédé, une pluralité de valeurs discrètes (Cdin1...Cdinj...Cdinp) prises par la conductivité du liquide de dialyse en amont de l'hémodialyseur 1, à des instants t1...tj...tn, est mise en mémoire dans l'unité de commande et de calcul 30. Deux instants successifs quelconques tj, tj+1 sont séparés par une même période d'échantillonnage Ts. Lorsque les variations de la conductivité sont commandées, ce sont de préférence les valeurs de conductivité correspondant à des signaux de commande qui sont mises en mémoire. En revanche, lorsque les variations de conductivité résultent du mode de production du liquide de dialyse, les valeurs (Cdin1...Cdinj...Cdinp) de conductivité qui sont mises en mémoire sont mesurées au moyen de la troisième sonde de conductivité 23.

Dans une troisième étape du procédé, une pluralité de valeurs discrètes (Cdout1...Cdoutj...Cdoutp) de la conductivité du liquide de dialyse est mesurée en aval de l'hémodialyseur 1, aux instants t1...tj...tn, au moyen de la quatrième sonde de conductivité 25, et est mise en mémoire dans l'unité de commande et de calcul 30. A chaque valeur de la conductivité Cdinj à l'instant instant t = j en aval de l'hémodialyseur correspond une valeur de la conductivité Cdoutj+n en amont de l'hémodialyseur à l'instant t = j+z, le décalage temporel entre ces deux valeurs étant égal au délai hydraulique T (c'est-à-dire z = T/Ts).

L'étape suivante du procédé est une étape de calcul. Pour chaque valeur (Cdinj) d'une série de m valeurs de la conductivité en amont du dialyseur, et à partir d'une valeur estimée initiale (D1) du paramètre (par exemple, la dialysance D) dont on cherche à déterminer la valeur réelle à tout moment, l'unité de calcul et de commande 30 calcule, au moyen du modèle mathématique, une valeur (Cd*outj+z) de la conductivité en aval de l'hémodialyseur 1 (dans la suite, le symbole * indique une valeur calculée). Chaque valeur (Cd*outj+z) calculée de la conductivité en aval pour l'instant t = j+z est alors comparée à la valeur (Cdoutj+z) mesurée en aval à l'instant t = j+z. Si le résultat de la comparaison indique que les valeurs calculées (Cd*outj+z) et les valeurs mesurées (Cdoutj+z) sont proches (si leur différence ou leur quotient est, par exemple, inférieur à un seuil prédéterminé), l'unité de calcul et de commande 30 affiche la valeur numérique D1 du paramètre D utilisée dans les calculs comme étant la valeur réelle instantanée du paramètre. Dans le cas contraire, l'unité de calcul 30 réitère les opérations précédentes avec une seconde, puis éventuellement une troisième, une quatrième, etc. valeur numérique D2, D3, D4 du paramètre D, jusqu'à ce que le résultat de la comparaison soit satisfaisant.

Quand le modèle mathématique utilisé est du premier ordre, une méthode particulièrement appropriée à la détermination de la dialysance D est la méthode des moindre carrés, dont on rappelle qu'elle consiste à sélectionner la valeur numérique (D1, D2,... Dn) de la dialysance D pour laquelle la somme des carrés des différences entre valeur mesurée et valeur calculée correspondante de la caractéristique en aval de l'hémodialyseur est minimale, soit:

$$\sum \; [Cdoutj+z - Cd*outj+z]^2$$

Conformément à l'invention, le procédé qui vient d'être décrit est continu:

- à tout moment, les m valeurs (Cdout1... Cdoutm) de la conductivité aval mesurées à partir desquelles les calculs sont effectués incluent la dernière ou l'une des dernières valeurs (Cdout1... Cdoutm) de la conductivité aval mesurées;
- le paramètre D dont on cherche à établir la valeur réelle est déterminé chaque fois qu'une nouvelle valeur de la conductivité aval (Cdoutj) est mesurée et mise en mémoire, ou, plus généralement toutes les fois qu'un nombre entier de nouvelles valeurs (Cdoutj) est mémorisé (par exemple toutes les deux ou trois valeurs).

Le nombre de valeurs m à partir duquel le paramètre D est déterminé est choisi en fonction de la période d'échantillonnage Ts, de façon que le durée totale d'acquisition de ces m valeurs soit suffisamment courte pour que l'on puisse considérer que la concentration ionique du sang reste constante durant cette durée d'acquisition.

A partir de la valeur réelle de la dialysance D, d'une valeur de la conductivité Cdinj fixée ou mesurée en amont de l'hémodialyseur 1, de la valeur correspondante de la conductivité Cdoutj+z mesurée en aval de l'hémodialyseur 1, et du débit Qd du liquide de dialyse, l'unité de calcul et de commande 30 peut calculer la concentration ionique équivalente du sang Cbin en appliquant la formule classique:

$$D = Qd \times \frac{Cdinj - Cdoutj+z}{Cbin - Cdinj}$$

L'unité de calcul et de commande 30 peut en outre calculer la clairance K réelle pour l'urée, à partir de la valeur réelle de la dialysance D, et à partir de tables de correspondance, préalablement mises en mémoire, entre la dialysance D pour le sodium et la clairance K pour l'urée.

Enfin, à partir de la clairance K réelle, de la durée écoulée du traitement t, et du volume V de répartition de l'urée dans le patient (qui dépend du poids moyen, du sexe et de l'âge), l'unité de calcul et de commande 30 peut aussi calculer la dose de dialyse administrée Kt/V.

Exemple 1:

Un premier exemple de modèle mathématique, au sens de l'invention, découle de l'équation différentielle suivante qui représente le transfert d'une substance ionisée (sodium) au travers de la membrane d'un hémodialyseur dans lequel sont mis en circulation, de part et d'autre de la membrane, le sang d'un patient et un liquide de dialyse:

$$\frac{dCdout(t)}{dt} = \frac{1}{\emptyset} [- Cdout(t) + (1-Dr) \times Cdin(t-T) + Dr \times Cbin(t)] \qquad (I)$$

avec Dr = D/Qd, où Qd est le débit de liquide de dialyse et D est la dialysance

Dans cette équation:

- Cdin(t) est la concentration en sodium dans le liquide de dialyse, en amont de l'hémodialyseur
- Cdout(t) est la concentration en sodium dans le liquide de dialyse, en aval de l'hémodialyseur
- Cbin(t) est la concentration en sodium dans le sang, en amont de l'hémodialyseur
- T est le délai hydraulique
- Ø est la constante de temps
- Dr est la dialysance relative

En partant de l'observation que, sur un intervalle de temps de l'ordre de quelques minutes, la concentration ionique du sang Cbin ne varie sensiblement pas, et en ne considérant que des valeurs discrètes prises par la conductivité en amont (Cbinj) et en aval (Cboutj) de l'hémodialyseur à des instants successifs t1...tj,tj+1...tm, l'équation (I) peut être réécrite de la façon suivante:

$$Cdoutj+z+1 = a \times Cdoutj+z + b \times Cdinj + c \qquad (II)$$

avec z = T/Ts, Ts étant la période d'échantillonnage de la conductivité du liquide de dialyse, la valeurs Cdoutj+z et Cdinj représentant donc la conductivité d'un même volume de liquide avant et après son passage dans l'hémodialyseur. Dans cet exemple on supposera que le délai hydraulique est connu.

Les coefficients a, b, c de l'équation (II) sont liés aux coefficients de l'équation différentielle (I) de la façon suivante:

$$\varnothing = - \frac{Ts}{\ln(a)} \qquad\qquad (III)$$

$$Dr = 1 - \frac{b}{1 - e^{-Ts/\varnothing}} \qquad\qquad (IV)$$

$$Cbin = \frac{c}{Dr \times (1 - e^{-Ts/\varnothing})} \qquad\qquad (V)$$

Conformément à l'invention, les équations (II) à (V) constituent un modèle mathématique qui peut être utilisé pour la mise en oeuvre d'un procédé de détermination de la dialysance D et de la concentration Cbin du sodium dans le sang pendant un traitement de dialyse.

Une première étape du procédé consiste à faire varier en permanence, autour d'une valeur moyenne, la conductivité du liquide de dialyse.

La valeur Cdin prise par la conductivité en amont du dialyseur (valeur mesurée ou valeur de consigne) est mise en mémoire régulièrement et de façon cumulative dans une mémoire de l'unité de calcul 30, qui contient donc en permanence une pluralité de valeurs discrètes de la conductivité (Cdin1...Cdinj...Cdinp) prises en amont de l'hémodialyseur 1 respectivement aux instants t1...tj...tp séparés par la période d'échantillonnage Ts.

De même, la valeur Cdout prise par la conductivité en aval du dialyseur (valeur mesurée) est mise en mémoire régulièrement et de façon cumulative dans une mémoire de l'unité de calcul 30, qui contient donc en permanence une pluralité de valeurs discrètes de la conductivité (Cdout1...Cdoutj...Cdoutp) prises en aval de l'hémodialyseur 1 respectivement aux instants t1...tj...tp séparés par la période d'échantillonnage Ts.

Pour déterminer la valeur de Cbin et de D à l'instant tp, l'unité de calcul est programmée pour poser, à partir d'une première série de m valeurs Cdin et d'une seconde série de m valeurs Cdout, une série d'équations:

$$Cd{*}outj+z+1 = [a \times Cd{*}outj+z + b \times Cdinj + c] + errj+z$$

dans laquelle errj+z est la différence entre la valeur calculée Cd*outj+z, au moyen de l'équation (II), de la conductivité pour l'instant j+z en aval de l'hémodialyseur et la valeur mesurée Cdoutj+z de la conductivité à l'instant j+z en aval de l'hémodialyseur.

Soit, sous forme de matricielle:

$$Z = H \times P + E$$

où

$$Z = \begin{bmatrix} Cd^*out1+z \\ Cd^*out2+z \\ \ldots\ldots \\ Cd^*outm+z-1 \\ Cd^*outm+z \end{bmatrix} \qquad H = \begin{bmatrix} Cd^*outz & Cdin0 & 1 \\ Cd^*out1+z & Cdin1 & 1 \\ \ldots\ldots & \ldots\ldots & 1 \\ Cd^*outm+z-2 & Cdinm-2 & 1 \\ Cd^*outm+z-1 & Cdinm-1 & 1 \end{bmatrix}$$

$$P = \begin{bmatrix} a \\ b \\ c \end{bmatrix} \qquad E = \begin{bmatrix} errz \\ err1+z \\ \ldots \\ errm+z-2 \\ errm+z-1 \end{bmatrix}$$

L'unité de calcul est programmée en outre pour mettre en oeuvre la méthode des minima carrés par laquelle il est possible de déterminer la matrice P pour laquelle la différence entre les valeurs calculées Cd*out et mesurées Cdout est la moindre, soit:

$$P = (H' \times H)^{-1} \times H \times Z$$

où H' est la matrice transposé de H.

Lorsque P est connue, c'est-à-dire les coefficients a, b, c, l'unité de calcul 30 calcule et affiche Cbin et D à partir des équations (III) à (V).

Le calcul de la variance V de la différence err = Cd*out-Cdout, selon la formule:

$$V = \frac{(Z - H \times P)^{-1} \times (Z - H \times P)}{m - 3}$$

fournit une indication sur la précision de la valeur de la dialysance D et de la concentration ionique du sang Cbin déterminées au moyen du procédé selon l'invention.

Conformément à l'invention, chaque fois qu'un nouveau couple de valeurs Cdinj, Cdoutj+z est mis en mémoire, l'unité de calcul 30 détermine une nouvelle valeur de la dialysance D et une nouvelle valeur de la concentration ionique du sang Cbin à partir des deux séries les plus récentes des m valeurs prises par la conductivité du liquide de dialyse en amont et en aval du dialyseur.

Exemple 2:

Dans cet exemple, le modèle mathématique est le même que précédemment, mais le délai hydraulique T n'est pas connu. Le procédé selon l'invention comprend alors une étape préliminaire de détermination du délai hydraulique qui consiste à exécuter de façon itérative les calculs qui viennent d'être décrits en utilisant à chaque fois une valeur numérique différente du délai hydraulique T. La valeur du délai hydraulique qui est retenue à l'issue de cette étape préliminaire est celle pour laquelle le variance V de la différence err = Cd*out - Cdout est la moindre.

Cette étape de détermination du délai hydraulique n'a naturellement pas besoin d'être réitérée chaque fois qu'un nouveau couple de valeurs de conductivité Cdinj, Cdoutj+z est mis en mémoire. Toutefois, chaque fois que le débit de liquide de dialyse est modifié, où si un nouvel hémodialyseur est utilisé en cours de séance, le délai hydraulique doit être calculé à nouveau.

Exemple 3:

L'unité de commande 30 impose une variation périodique (par exemple sinusoïdale) à la conductivité du liquide de dialyse préparé par le générateur de liquide de dialyse 11.

Conformément à l'invention, la valeur Cdin prise par la conductivité en amont du dialyseur (valeur mesurée ou valeur de consigne) est mise en mémoire régulièrement et de façon cumulative dans une mémoire de l'unité de calcul 30, qui contient donc en permanence une pluralité de valeurs discrètes de la conductivité (Cdin1...Cdinj...Cdinp) prises en amont de l'hémodialyseur 1 respectivement aux instants t1...tj...tp séparés par la période d'échantillonnage Ts.

De même, la valeur Cdout prise par la conductivité en aval du dialyseur (valeur mesurée) est mise en mémoire régulièrement et de façon cumulative dans une mémoire de l'unité de calcul 30, qui contient donc en permanence une pluralité de valeurs discrètes de la conductivité (Cdout1...Cdoutj...Cdoutp) prises en aval de l'hémodialyseur 1 respectivement aux instants t1...tj...tp séparés par la période d'échantillonnage Ts.

A partir d'une première série de m valeurs de la conductivité en amont de l'hémodialyseur, l'unité de calcul 30 est programmée pour déterminer l'amplitude |Cdin| de la variation de la conductivité en amont de l'hémodialyseur. De même, à partir d'une seconde série de m valeurs de la conductivité en aval de l'hémodialyseur, l'unité de calcul 30 est programmée pour déterminer l'amplitude |Cdout| de la variation de la conductivité en aval de l'hémodialyseur.

Pourvu que la période de la variation périodique imposée à la conductivité soit choisie suffisamment grande par rapport à la constante de temps Ø du système, la dialysance relative Dr peut être calculée simplement au moyen de la formule suivante:

$$Dr = \frac{D}{Qd} = 1 - \frac{|Cdout|}{|Cdin|}$$

Pour calculer la concentration ionique du sang Cbin, l'unité de calcul 30 détermine préalablement, à partir des m dernières valeurs de conductivité enregistrée, la conductivité moyenne CdinM en amont de l'hémodialyseur et la conductivité moyenne CdoutM en aval de l'hémodialyseur, puis elle applique la formule suivante:

$$Cbin = \frac{1}{Dr} \times CdoutM - \frac{(1 - Dr)}{Dr} \times CdinM$$

Un intérêt de ce deuxième mode de réalisation de l'invention est qu'il n'exige à aucun moment de connaître le délai hydraulique T.

## Revendications

1. Dispositif de détermination continue d'un paramètre (D, Cbin, K, Kt/v) significatif de l'efficacité d'un traitement extracorporel du sang consistant à faire circuler de part et d'autre de la membrane semi-perméable (4) d'un échangeur (1) à membrane le sang d'un patient et un liquide de traitement, ce dispositif comportant:

   • des moyens (22, 26) pour faire circuler dans l'échangeur (1) un liquide de traitement ayant une caractéristique (Cd) liée à l'efficacité du traitement;
   • des moyens (16, 17, 19, 20 ; 19, 30) pour provoquer une succession de variations de la caractéristique (Cd) en amont de l'échangeur (1);
   • des moyens (30) pour mettre en mémoire en continu une pluralité de valeurs (Cdin1...Cdinj...Cdinp) de la caractéristique (Cd) en amont de l'échangeur (1);
   • des moyens (25, 30) pour mesurer et mettre en mémoire en continu une pluralité de valeurs (Cdout1...Cdoutj...Cdoutp) prises par la caractéristique (Cd) en aval de l'échangeur (1) en réponse aux variations de la caractéristique (Cd) provoquées en amont de l'échangeur (1); **caractérisé en ce que**
   • des moyens de calcul (30) pour calculer, chaque fois qu'un nombre déterminé de nouvelles valeurs (Cdoutj) de la caractéristique (Cd) en aval de l'échangeur (1) a été mémorisé, un paramètre (D, Cbin, K, Kt/v) significatif de l'efficacité du traitement extracorporel du sang, à partir d'une première série de valeurs (Cdinj) de la caractéristique (Cd) en amont de l'échangeur (1), d'une deuxième série de valeurs (Cdoutj) de la caractéristique (Cd) en aval de l'échangeur (1), et au moyen d'un modèle mathématique de l'influence de la caractéristique (Cd) sur l'efficacité du traitement, le modèle mathématique ayant au moins un coefficient constitué par un paramètre (D, Cbin) significatif de l'efficacité du traitement extracorporel du sang.

2. Dispositif selon la revendication 1, **caractérisé en ce que** les moyens de calcul (30) calculent le paramètre (D, Cbin, K, Kt/v) significatif de l'efficacité du traitement extracorporel du sang chaque fois qu'une nouvelle valeur (Cdoutj) de la caractéristique (Cd) en aval de l'échangeur (1) à été mémorisée.

3. Dispositif selon une des revendications 1 et 2, **caractérisé en ce que** la deuxième série de valeurs (Cdoutj) de la caractéristique (Cd) en aval de l'échangeur (1) comprend la dernière valeur mise en mémoire.

4. Dispositif selon une des revendications 1 à 3, **caractérisé en ce que** la deuxième série de valeurs (Cdoutj) de la caractéristique (Cd) en aval de l'échangeur (1) comprend un nombre déterminé de valeurs successives.

5. Dispositif selon la revendication 1, **caractérisé en ce que** les moyens de calcul (30) établissent une correspondance entre chaque valeur (Cdoutj+z) de la deuxième série de valeurs et une valeur (Cdinj) de la première série de valeur, la valeur (Cdoutj+z) de la deuxième série de valeurs étant décalée dans le temps par rapport à la valeur (Cdinj) correspondante de la première série de valeurs d'un délai hydraulique (T) égal au temps mis par un échantillon de liquide pour s'écouler dans un circuit (9, 10) de liquide de traitement connecté à l'échangeur (1), entre un point situé en amont de l'échangeur (1) et un point situé en aval de l'échangeur (1).

6. Dispositif selon la revendication 5, **caractérisé en ce que** les moyens de calcul (30) déterminent la valeur du délai hydraulique (T).

7. Dispositif selon la revendication 6, **caractérisé en ce que** le délai hydraulique (T) est l'un des coefficients du modèle mathématique et **en ce que**, pour déterminer la valeur du délai hydraulique (T), les moyens de calcul (30):

   - calculent au moyen du modèle mathématique, pour chaque valeur (Cdinj) de la première série de valeurs, une valeur (Cd*outj+z) correspondante de la caractéristique (Cd) en aval de l'échangeur (1); et
   - déterminent la valeur optimale du délai hydraulique (T) pour laquelle la correspondance entre les valeurs calculées (Cd*outj+z) de la caractéristique (Cd) en aval de l'échangeur (1) et les valeurs mesurées (Cdoutj+z) correspondantes de la caractéristique (Cd) en aval de l'échangeur (1) est la plus exacte.

8. Dispositif selon une des revendications 5 à 7, **caractérisé en ce que**, pour calculer un paramètre (D, Cbin) significatif de l'efficacité du traitement extracorporel du sang, les moyens de calcul (30):

   - calculent au moyen du modèle mathématique, pour chaque valeur (Cdinj) de la première série de valeurs, une valeur (Cd*outj+z) correspondante de la caractéristique (Cd) en aval de l'échangeur (1); et
   - déterminent la valeur optimale du paramètre (D, Cbin) pour laquelle la correspondance entre les valeurs calculées (Cd*outj+z) de la caractéristique (Cd) en aval de l'échangeur (1) et les valeurs mesurées (Cdoutj+z) correspondantes de la caractéristique (Cd) en aval de l'échangeur (1) est la plus exacte.

9. Dispositif selon la revendication 8, **caractérisé en ce que** le modèle mathématique est linéaire et **en ce que**, pour déterminer la valeur optimale du paramètre (D, Cbin), les moyens de calcul (30) déterminent la valeur du paramètre (D, Cbin) pour laquelle la somme des carrés des différences entre valeurs mesurées (Cdoutj+z) et valeurs calculées (Cd*outj+z) correspondantes de la caractéristique (Cd) en aval de l'échangeur est la moindre.

10. Dispositif selon une des revendications 1 à 9, **caractérisé en ce que** les moyens (19, 30) pour provoquer une succession de variations de la caractéristique (Cd) en amont de l'échangeur (1) règlent la caractéristique (Cd) en fonction de l'évolution d'un paramètre d'un dispositif (29) destiné à la mise en oeuvre du traitement et/ou d'un paramètre du patient, de façon que ce paramètre reste contenu à l'intérieur d'une fourchette de valeurs admissibles.

11. Dispositif selon la revendication 10, **caractérisé en ce que** le paramètre du patient est la variation relative du volume du sang du patient.

**12.** Dispositif selon une des revendications 1 à 9, **caractérisé en ce que**, en vue de faire varier la caractéristique (Cd) en amont de l'échangeur (1), les moyens de mise en mémoire (30) contiennent une règle de variation de la caractéristique en amont de l'échangeur (1) mise en mémoire préalablement.

**13.** Dispositif selon la revendication 12, **caractérisé en ce que** les moyens (19, 30) pour provoquer une succession de variations de la caractéristique (Cd) en amont de l'échangeur (1) provoquent l'alternance régulière d'une augmentation et d'une diminution de la caractéristique (Cd), d'une quantité déterminée.

**14.** Dispositif selon une des revendications 1 à 9, **caractérisé en ce qu'**il comporte en outre des moyens (11) pour préparer le liquide de traitement et **en ce que** la variation de la caractéristique (Cd) en amont de l'échangeur (1) est inhérente aux perturbations liées à la préparation du liquide de traitement.

**15.** Dispositif selon une des revendications 1 à 14, **caractérisé en ce qu'**il comporte en outre des moyens (23) pour mesurer les valeurs (Cdinj) de la caractéristique (Cd) en amont de l'échangeur (1).

**16.** Dispositif selon une des revendications 1 à 15, **caractérisé en ce que** la caractéristique du liquide de traitement liée à l'efficacité du traitement est une caractéristique (Cd) du liquide de traitement liée aux échanges au travers de la membrane de l'échangeur (1) et **en ce que** le modèle mathématique de l'influence de la caractéristique (Cd) sur l'efficacité du traitement est un modèle mathématique des échanges au travers de la membrane de l'échangeur (1).

**17.** Dispositif selon la revendication 16, **caractérisé en ce que** la caractéristique (Cd) du liquide de traitement est liée aux échanges d'ions, de solutés ou aux échanges thermiques au travers de la membrane de l'échangeur (1).

**18.** Dispositif selon la revendication 17, **caractérisé en ce que** le paramètre significatif de l'efficacité d'un traitement extracorporel du sang est la dialysance (D) et **en ce que** le modèle mathématique est défini par les équations suivantes:

$$Cdout_{j+z+1} = a \times Cdout_{j+z} + b \times Cdinj + c$$

$$\varnothing = - \frac{Ts}{\ln(a)}$$

$$Dr = 1 - \frac{b}{1 - e^{-Ts/\varnothing}}$$

$$Cbin = \frac{c}{Dr \times (1 - e^{-Ts/\varnothing})}$$

dans lesquelles:

- Cdinj est la concentration en sodium dans le liquide de traitement, en amont de l'échangeur (1);
- Cdoutj est la concentration en sodium dans le liquide de traitement, en aval de l'échangeur (1);
- Ts est la période d'échantillonnage;
- Ø est la constante de temps;

- Dr = D/Qd est la dialysance relative, Qd étant le débit de liquide de traitement et D étant la dialysance.

**19.** Dispositif selon la revendication 17, **caractérisé en ce que** les moyens (19, 30) pour provoquer une succession de variations de la caractéristique (Cd) en amont de l'échangeur (1) imposent à la caractéristique (Cd) du liquide de dialyse une variation périodique, et **en ce que** les moyens de calcul (30) calculent l'amplitude |Cdin| de la variation de la conductivité en amont de l'échangeur (1) à partir de la première série de valeurs (Cdinj) et l'amplitude |Cdout| de la variation de la conductivité en aval de l'échangeur (1) à partir de la deuxième série de valeurs (Cdoutj).

**20.** Dispositif selon la revendication 19, **caractérisé en ce que** le paramètre significatif de l'efficacité d'un traitement extracorporel du sang est la dialysance (D) et **en ce que** le modèle mathématique est défini par l'équation suivante:

$$Dr = \frac{D}{Qd} = 1 - \frac{|Cdout|}{|Cdin|}$$

dans laquelle Qd est le débit du liquide de traitement.

**21.** Dispositif selon la revendication 20, **caractérisé en ce que** les moyens de calcul (30) calculent en outre la conductivité moyenne CdinM en amont de l'échangeur (1) à partir de la première série de valeurs (Cdinj) et la conductivité moyenne CdoutM en aval de l'échangeur (1) à partir de la deuxième série de valeurs (Cdoutj).

**22.** Dispositif selon la revendication 21, **caractérisé en ce que** le paramètre significatif de l'efficacité d'un traitement extracorporel du sang est la concentration ionique Cbin du sang en amont de l'échangeur (1) et **en ce que** le modèle mathématique est défini par l'équation suivante:

$$Cbin = \frac{1}{Dr} \times CdoutM - \frac{(1 - Dr)}{Dr} \times CdinM$$

**Patentansprüche**

**1.** Vorrichtung zur kontinuierlichen Ermittlung eines maßgeblichen Parameters (D, Cbin, K, Kt/v) der Wirksamkeit einer extrakorporalen Blutbehandlung, bestehend aus Zirkulieren des Bluts eines Patienten sowie einer Behandlungsflüssigkeit von einer Seite zur anderen der halbdurchlässigen Membran (4) eines Membrantauschers (1), diese Vorrichtung umfassend:

• Mittel (22, 26) zum Zirkulieren einer Behandlungsflüssigkeit, die eine Charakteristik (Cd) aufweist, die mit der Behandlungswirksamkeit verbunden ist, im Tauscher (1);
• Mittel (16, 17, 19, 20; 19, 30) zum Bewirken einer Abfolge von Variationen der Charakteristik (Cd) stromaufwärts des Tauschers (1);
• Mittel (30) zum kontinuierlichen Speichern einer Mehrzahl von Werten (Cdin1 ... Cdinj ... Cdinp) der Charakteristik (Cd) stromaufwärts des Tauschers (1);
• Mittel (25, 30) zum kontinuierlichen Messen und Speichern einer Mehrzahl von Werten (Cdout1 ... Cdoutj ... Cdoutp), die durch die Charakteristik (Cd) stromabwärts des Tauschers (1) in Reaktion auf Variationen der Charakteristik (Cd), die stromaufwärts des Tauschers (1) bewirkt werden; **gekennzeichnet durch**
• Berechnungsmittel (30) zum Berechnen eines maßgeblichen Parameters (D, Cbin, K, Kt/v) der Wirksamkeit der extrakorporalen Blutbehandlung, jedes Mal, wenn eine vorbestimmte Anzahl neuer Werte (Cdoutj) der Charakteristik (Cd) stromabwärts des Tauschers (1) gespeichert wurde, ausgehend von einer ersten Werteserie (Cdinj) der Charakteristik (Cd) stromaufwärts des Tauschers (1), von einer zweiten Werteserie (Cdoutj) der Charakteristik (Cd) stromabwärts des Tauschers (1) und mittels eines mathematischen Modells des Einflusses der Charakteristik (Cd) auf die Wirksamkeit der Behandlung, wobei das mathematische Modell mindestens einen Koeffizienten aufweist, bestehend aus einem maßgeblichen Parameter (D, Cbin) der Wirksamkeit der extrakorporalen Blutbehandlung.

**2.** Vorrichtung nach Anspruch 1, **dadurch gekennzeichnet, dass** das Berechnungsmittel (30) den maßgeblichen Parameter (D, Cbin, K, Kt/v) der Wirksamkeit der extrakorporalen Blutbehandlung, jedes Mal berechnet, wenn ein neuer Wert (Cdoutj) der Charakteristik (Cd) stromabwärts des Tauschers (1) gespeichert wurde.

**3.** Vorrichtung nach einem der Ansprüche 1 und 2, **dadurch gekennzeichnet, dass** die zweite Werteserie (Cdoutj) der Charakteristik (Cd) stromabwärts des Tauschers (1) den letzten gespeicherten Wert umfasst.

**4.** Vorrichtung nach einem der Ansprüche 1 bis 3, **dadurch gekennzeichnet, dass** die zweite Werteserie (Cdoutj) der Charakteristik (Cd) stromabwärts des Tauschers (1) eine vorbestimmte Anzahl an aufeinanderfolgenden Werten umfasst.

**5.** Vorrichtung nach Anspruch 1, **dadurch gekennzeichnet, dass** das Berechnungsmittel (30) eine Übereinstimmung zwischen jedem Wert (Cdoutj+z) der zweiten Werteserie und einem Wert (Cdinj) der ersten Werteserie feststellt, wobei der Wert (Cdoutj+z) der zweiten Werteserie im Vergleich zum entsprechenden Wert (Cdinj) der ersten Werteserie einer hydraulischen Verzögerung (T) zeitlich versetzt ist, entsprechend der Zeit, die eine Flüssigkeitsprobe benötigt, um in einem Kreislauf (9, 10) der Behandlungsflüssigkeit, verbunden mit dem Tauscher (1), zwischen einem Punkt stromaufwärts des Tauschers (1) und einem Punkt stromabwärts des Tauschers (1) zu fließen.

**6.** Vorrichtung nach Anspruch 5, **dadurch gekennzeichnet, dass** das Berechnungsmittel (30) den Wert der hydraulischen Verzögerung (T) ermittelt.

**7.** Vorrichtung nach Anspruch 6, **dadurch gekennzeichnet, dass** die hydraulische Verzögerung (T) einer der Koeffizienten des mathematischen Modells ist und dadurch, dass, zur Ermittlung des Werts der hydraulischen Verzögerung (T), das Berechnungsmittel (30):

- mittels des mathematischen Modells für jeden Wert (Cdinj) der ersten Werteserie einen entsprechenden Wert (Cd*outj+z) der Charakteristik (Cd) stromabwärts des Tauschers (1) berechnet und
- den optimalen Wert der hydraulischen Verzögerung (T) ermittelt, für den die Übereinstimmung zwischen den berechneten Werten (Cd*outj+z) der Charakteristik (Cd) stromabwärts des Tauschers (1) und den entsprechenden stromabwärts des Tauschers (1) gemessenen Werten (Cdoutj+z) der Charakteristik (Cd) die genaueste ist.

**8.** Vorrichtung nach Anspruch 5 bis 7, **dadurch gekennzeichnet, dass**, zur Berechnung eines maßgeblichen Parameters (D, Cbin) der Wirksamkeit der extrakorporalen Blutbehandlung, das Berechnungsmittel (30):

- mittels des mathematischen Modells für jeden Wert (Cdinj) der ersten Werteserie einen entsprechenden Wert (Cd*outj+z) der Charakteristik (Cd) stromabwärts des Tauschers (1) berechnet; und
- den optimalen Wert des Parameters (D, Cbin) ermittelt, für den die Übereinstimmung zwischen den berechneten Werten (Cd*outj+z) der Charakteristik (Cd) stromabwärts des Tauschers (1) und den entsprechenden stromabwärts des Tauschers (1) gemessenen Werten (Cdoutj+z) der Charakteristik (Cd) die genaueste ist.

**9.** Vorrichtung nach Anspruch 8, **dadurch gekennzeichnet, dass** das mathematische Modell linear ist und dadurch, dass das Berechnungsmittel (30), um den optimalen Wert des Parameters (D, Cbin) zu ermitteln, den Wert des Parameters (D, Cbin) ermittelt, für den die Summe der Quadrate der Differenzen zwischen den gemessenen Werten (Cdoutj+z) und den entsprechenden berechneten Werten (Cd*outj+z) der Charakteristik (Cd) stromabwärts des Tauschers die kleinste ist.

**10.** Vorrichtung nach einem der Ansprüche 1 bis 9, **dadurch gekennzeichnet, dass** das Mittel (19, 30) zum Bewirken einer Abfolge von Variationen der Charakteristik (Cd) stromaufwärts des Tauschers (1) die Charakteristik (Cd) abhängig von der Entwicklung eines Parameters einer Vorrichtung (29), bestimmt zur Ausführung der Behandlung, und/oder eines Parameters des Patienten regeln, sodass dieser Parameter innerhalb eines zulässigen Wertebereichs bleibt.

**11.** Vorrichtung nach Anspruch 10, **dadurch gekennzeichnet, dass** der Parameter des Patienten die entsprechende Variation des Blutvolumens des Patienten darstellt.

**12.** Vorrichtung nach einem der Ansprüche 1 bis 9, **dadurch gekennzeichnet, dass** das Mittel zum Speichern (30) eine Regel zur Variation der Charakteristik stromaufwärts des Tauschers (1), die zuvor gespeichert wurde, enthält, um die Charakteristik (Cd) stromaufwärts des Tauschers (1) variieren zu lassen.

**13.** Vorrichtung nach Anspruch 12, **dadurch gekennzeichnet, dass** das Mittel (19, 30) zum Bewirken einer Abfolge von Variationen der Charakteristik (Cd) stromaufwärts des Tauschers (1) den regelmäßigen Wechsel einer Erhöhung und einer Reduzierung der Charakteristik (Cd) um eine vorbestimmte Menge bewirkt.

**14.** Vorrichtung nach einem der Ansprüche 1 bis 9, **dadurch gekennzeichnet, dass** sie weiter ein Mittel (11) zum Vorbereiten der Behandlungsflüssigkeit umfasst, und dadurch, dass die Variation der Charakteristik (Cd) stromaufwärts des Tauschers (1) mit den Störungen zusammenhängt, die mit der Vorbereitung der Behandlungsflüssigkeit verbunden sind.

**15.** Vorrichtung nach einem der Ansprüche 1 bis 14, **dadurch gekennzeichnet, dass** sie weiter ein Mittel (23) zum Messen der Werte (Cdinj) der Charakteristik (Cd) stromaufwärts des Tauschers (1) umfasst.

**16.** Vorrichtung nach einem der Ansprüche 1 bis 15, **dadurch gekennzeichnet, dass** es sich bei der Charakteristik der Behandlungsflüssigkeit, die mit der Wirksamkeit der Behandlung verbunden ist, um eine Charakteristik (Cd) der Behandlungsflüssigkeit handelt, verbunden mit Tauschvorgängen über die Membran des Tauschers (1), und dadurch, dass es sich beim mathematischen Modell zur Beeinflussung der Charakteristik (Cd) bezüglich der Wirksamkeit der Behandlung um ein mathematisches Modell der Tauschvorgänge durch die Membran des Tauschers (1) handelt.

**17.** Vorrichtung nach Anspruch 16, **dadurch gekennzeichnet, dass** die Charakteristik (Cd) der Behandlungsflüssigkeit mit dem Tausch von Ionen, gelösten Stoffen oder dem Wärmetausch durch die Membran des Tauschers (1) verbunden ist.

**18.** Vorrichtung nach Anspruch 17, **dadurch gekennzeichnet, dass** es sich beim Parameter, der maßgeblich für die Wirksamkeit der extrakorporalen Blutbehandlung ist, um die Dialysierfähigkeit (D) handelt und dadurch, dass das mathematische Modell durch folgende Gleichungen definiert ist:

$$Cdout_{j+z+1} = a \times Cdout_{j+z} + b \times Cdin_j + c$$

$$\emptyset = -\frac{Ts}{\ln(a)}$$

$$Dr = 1 - \frac{b}{1 - e^{-Ts/\emptyset}}$$

$$Cbin = \frac{c}{Dr \times (1 - e^{-Ts/\emptyset})}$$

wobei:

- Cdinj die Natriumkonzentration in der Behandlungsflüssigkeit stromaufwärts des Tauschers (1) ist;
- Cdoutj die Natriumkonzentration in der Behandlungsflüssigkeit stromabwärts des Tauschers (1) ist;
- Ts die Probenahmezeit ist;
- 0 die Zeitkonstante ist;
- Dr = D/Qd die entsprechende Dialysierfähigkeit ist, wobei Qd die Durchflussmenge an Behandlungsflüssigkeit und D die Dialysierfähigkeit ist.

**19.** Vorrichtung nach Anspruch 17, **dadurch gekennzeichnet, dass** das Mittel (19, 30) zum Bewirken einer Abfolge an Variationen der Charakteristik (Cd) stromaufwärts des Tauschers (1) der Charakteristik (Cd) der Dialyseflüssigkeit eine periodische Variation vorschreibt, und dadurch, dass das Berechnungsmittel (30) die Amplitude (Cdin) der Variation der Leitfähigkeit stromaufwärts des Tauschers (1) berechnet, ausgehend von der ersten Werteserie (Cdinj), sowie die Amplitude (Cdout) der Variation der Leitfähigkeit stromabwärts des Tauschers (1), ausgehend von der zweiten Werteserie (Cdoutj).

**20.** Vorrichtung nach Anspruch 19, **dadurch gekennzeichnet, dass** es sich beim maßgeblichen Parameter der Wirksamkeit der extrakorporalen Blutbehandlung um die Dialysierfähigkeit (D) handelt und dadurch, dass das mathematische Modell durch folgende Gleichung definiert ist:

$$Dr = \frac{D}{Qd} = 1 - \frac{|Cdout|}{|Cdin|}$$

wobei Qd die Durchflussmenge der Behandlungsflüssigkeit ist.

**21.** Vorrichtung nach Anspruch 20, **dadurch gekennzeichnet, dass** das Berechnungsmittel (30) ferner die mittlere Leitfähigkeit CdinM stromaufwärts des Tauschers (1) berechnet, ausgehend von der ersten Werteserie (Cdinj), und die mittlere Leitfähigkeit CdoutM stromabwärts des Tauschers (1), ausgehend von der zweiten Werteserie (Cdoutj).

**22.** Vorrichtung nach Anspruch 21, **dadurch gekennzeichnet, dass** es sich beim maßgeblichen Parameter der Wirksamkeit der extrakorporalen Blutbehandlung um die Ionenkonzentration Cbin des Bluts stromaufwärts des Tauschers (1) handelt und dadurch, dass das mathematische Modell durch folgende Gleichung definiert ist:

$$Cbin = \frac{1}{Dr} \times CdoutM - \frac{(1 - Dr)}{Dr} \times CdinM$$

**Claims**

**1.** Device for continuous determination of a significant parameter (D, Cbin, K, Kt/v) of the efficacy of extracorporeal blood treatment consisting of circulating on one side and on the other side of the semipermeable membrane (4) of a membrane exchanger (1) the blood of a patient and a treatment liquid, this device comprising:

   • means (22, 26) for circulating in the exchanger (1) a treatment liquid having a characteristic (Cd) linked to the efficacy of the treatment;
   • means (16, 17, 19, 20 ; 19, 30) for causing a succession of variations of the characteristic (Cd) upstream of the exchanger (1);
   • means (30) for continuously storing a plurality of values (Cdin1...Cdinj ...Cdinp) of the characteristic (Cd) upstream of the exchanger (1);
   • means (25, 30) for measuring and storing continuously a plurality of values (Cdout1...Cdoutj ...Cdoutp) taken by the characteristic (Cd) downstream of the exchanger (1) in response to variations in the characteristic (Cd) caused upstream of the exchanger (1); **characterised by**
   • calculating means (30) for calculating, each time that a set number of new values (Cdoutj) of the characteristic (Cd) downstream of the exchanger (1) has been stored, a significant parameter (D, Cbin, K, Kt/v) of the efficacy of the extracorporeal blood treatment, starting from a first series of values (Cdinj) of the characteristic (Cd) upstream of the exchanger (1), of a second series of values (Cdoutj) of the characteristic (Cd) downstream of the exchanger (1), and by means of a mathematical model of the influence of the characteristic (Cd) on the efficacy of the treatment, the mathematical model having at least one coefficient consisting of a significant parameter (D, Cbin) of the efficacy of the extracorporeal blood treatment.

**2.** Device according to claim 1, **characterised in that** the calculating means (30) calculates the significant parameter (D, Cbin, K, Kt/v) of the efficacy of the extracorporeal blood treatment each time that a new value (Cdoutj) of the characteristic (Cd) downstream of the exchanger (1) has been stored.

**3.** Device according to any one of claims 1 and 2, **characterised in that** the second series of values (Cdoutj) of the characteristic (Cd) downstream of the exchanger (1) comprises the last stored value.

**4.** Device according to any one of claims 1 to 3, **characterised in that** the second series of values (Cdoutj) of the characteristic (Cd) downstream of the exchanger (1) comprises a set number of successive values.

**5.** Device according to claim 1, **characterised in that** the calculating means (30) establishes a correspondence between each value (Cdoutj+z) of the second series of values and a value (Cdinj) of the first series of values, the value (Cdoutj+z) of the second series of values being shifted over time in relation to the corresponding value (Cdinj) of the first series of values of a hydraulic delay (T) that is the same as the time taken for a sample of liquid to flow in a circuit (9, 10) of treatment liquid connected to the exchanger (1), between a point situated upstream of the exchanger (1) and a point situated downstream of the exchanger (1).

**6.** Device according to claim 5, **characterised in that** the calculating means (30) determines the value of the hydraulic delay (T).

**7.** Device according to claim 6, **characterised in that** the hydraulic delay (T) is one of the coefficients of the mathematical model and **in that**, to determine the value of the hydraulic delay (T), the calculating means (30):

- calculates by means of the mathematical model, for each value (Cdinj) of the first series of values, a corresponding value (Cd*outj+z) of the characteristic (Cd) downstream of the exchanger (1); and
- determines the optimum value of the hydraulic delay (T) for which the correspondence between the calculated values (Cd*outj+z) of the characteristic (Cd) downstream of the exchanger (1) and the corresponding measured values (Cdoutj+z) of the characteristic (Cd) downstream of the exchanger (1) is the most precise one.

**8.** Device according to any one of claims 5 to 7, **characterised in that**, for calculating a significant parameter (D, Cbin) of the efficacy of the extracorporeal blood treatment, the calculating means (30):

- calculates by means of the mathematical model, for each value (Cdinj) of the first series of values, a corresponding value (Cd*outj+z) of the characteristic (Cd) downstream of the exchanger (1); and
- determines the optimum value of the parameter (D, Cbin) for which the correspondence between the calculated values (Cd*outj+z) of the characteristic (Cd) downstream of the exchanger (1) and the corresponding measured values (Cdoutj+z) of the characteristic (Cd) downstream of the exchanger (1) is the most precise one.

**9.** Device according to claim 8, **characterised in that** the mathematical model is linear and **in that**, to determine the optimal value of the parameter (D, Cbin), the calculating means (30) determines the value of the parameter (D, Cbin) for which the sum of the squares of the differences between measured values (Cdoutj+z) and corresponding calculated values (Cd*outj+z) of the characteristic (Cd) downstream of the exchanger is the smallest one.

**10.** Device according to any one of claims 1 to 9, **characterised in that** the means (19, 30) for causing a succession of variations of the characteristic (Cd) upstream of the exchanger (1) manages the characteristic (Cd) according to the evolution of a parameter of a device (29) intended for starting up the treatment and/or of a parameter of the patient, such that this parameter remains within a range of permissible values.

**11.** Device according to claim 10, **characterised in that** the parameter of the patient is the corresponding variation of the volume of the blood of the patient.

**12.** Device according to any one of claims 1 to 9, **characterised in that**, in order to vary the characteristic (Cd) upstream of the exchanger (1), the storage means (30) contains a variation rule of the characteristic upstream of the exchanger (1) that is stored beforehand.

**13.** Device according to claim 12, **characterised in that** the means (19, 30) for causing a succession of variations of the characteristic (Cd) upstream of the exchanger (1) cause the regular alternation of an increase and of a decrease of the characteristic (Cd), by a set quantity.

14. Device according to any one of claims 1 to 9, **characterised in that** it further comprises means (11) for preparing the treatment liquid and **in that** the variation of the characteristic (Cd) upstream of the exchanger (1) is inherent to the perturbations linked to the preparation of the treatment liquid.

15. Device according to any one of claims 1 to 14, **characterised in that** it further comprises means (23) for measuring the values (Cdinj) of the characteristic (Cd) upstream of the exchanger (1).

16. Device according to any one of claims 1 to 15, **characterised in that** the characteristic of the treatment liquid linked to the efficacy of the treatment is a characteristic (Cd) of the treatment liquid linked to the exchanges through the membrane of the exchanger (1) and **in that** the mathematical model of the influence of the characteristic (Cd) on the efficacy of the treatment is a mathematical model of the exchanges through the membrane of the exchanger (1).

17. Device according to claim 16, **characterised in that** the characteristic (Cd) of the treatment liquid is linked to the exchanges of ions, of solutes, or of thermal exchanges through the membrane of the exchanger (1).

18. Device according to claim 17, **characterised in that** the significant parameter of the efficacy of an extracorporeal blood treatment is the dialysance (D) and **in that** the mathematical model is defined by the following equations:

$$Cdout_{j+z+1} = a \times Cdout_{j+z} + b \times Cdinj + c$$

$$\varnothing = - \frac{Ts}{\ln(a)}$$

$$Dr = 1 - \frac{b}{1 - e^{-Ts/\varnothing}}$$

$$Cbin = \frac{c}{Dr \times (1 - e^{-Ts/\varnothing})}$$

in which:

- Cdinj is the sodium concentration in the treatment liquid, upstream of the exchanger (1);
- Cdoutj is the sodium concentration in the treatment liquid, downstream of the exchanger (1);
- Ts is the sampling period;
- $\varnothing$ is the time constant;
- Dr = D/Qd is the corresponding dialysance, Qd being the flow of treatment liquid and D being the dialysance.

19. Device according to claim 17, **characterised in that** the means (19, 30) for causing a succession of variations of the characteristic (Cd) upstream of the exchanger (1) imposes on the characteristic (Cd) of the dialysis liquid a periodic variation, and **in that** the calculating means (30) calculates the extent |cdin| of the variation of the conductivity upstream of the exchanger (1) starting from the first series of values (Cdinj) and the extent |Cdout| of the variation of the conductivity downstream of the exchanger (1) starting from the second series of values (Cdoutj).

20. Device according to claim 19, **characterised in that** the significant parameter of the efficacy of extracorporeal blood treatment is the dialysance (D) and **in that** the mathematical model is defined by the following equation:

$$Dr = \frac{D}{Qd} = 1 - \frac{|Cdout|}{|Cdin|}$$

in which Qd is the flow of the treatment liquid.

21. Device according to claim 20, **characterised in that** the calculating means (30) further calculates the average conductivity CdinM upstream of the exchanger (1) starting from the first series of values (Cdinj) and the average conductivity CdoutM downstream of the exchanger (1) starting from the second series of values (Cdoutj).

22. Device according to claim 21, **characterised in that** the significant parameter of the efficacy of extracorporeal blood treatment is the ionic concentration Cbin of the blood upstream of the exchanger (1) and **in that** the mathematical model is defined by the following equation:

$$Cbin = \frac{1}{Dr} \times CdoutM - \frac{(1 - Dr)}{Dr} \times CdinM$$

Fig. 1

UNITE
DE
COMMANDE

30

31

32

EP 1 108 438 B1

20

11

12  17  20  21  22  9  23

Eau

19  14

18

15  13  16

3  1

4  2

7

8

25

27  26

29  10

5  6

24

## RÉFÉRENCES CITÉES DANS LA DESCRIPTION

**Documents brevets cités dans la description**

- EP 0547025 A
- EP 0658352 A
- EP 0920877 A

**Littérature non-brevet citée dans la description**

- **GOTCH FA ; SARGENT SA.** A mechanistic analysis of the National Cooperative Dialysis Study (NCDS. *Kidney int,* 1985, vol. 28, 526-34